(19) Europäisches Patentamt
European Patent Office
Office européen des brevets

(11) Publication number: **0 168 769**
**A2**

# (12) EUROPEAN PATENT APPLICATION

(21) Application number: 85108598.5

(22) Date of filing: 11.07.85

(51) Int. Cl.⁴: C 07 K 5/06

(30) Priority: 16.07.84 US 631132

(43) Date of publication of application: 22.01.86
Bulletin 86/4

(84) Designated Contracting States: CH DE FR GB IT LI NL

(71) Applicant: MERCK & CO. INC., 126, East Lincoln Avenue
P.O. Box 2000, Rahway New Jersey 07065 (US)

(72) Inventor: Blacklock, Thomas J., 493 Valley Road, Clark
New Jersey 07066 (US)
Inventor: Shuman, Richard F., 915 Willow Grove Road,
Westfield New Jersey 07090 (US)

(74) Representative: Abitz, Walter, Dr.-Ing. et al, Abitz, Morf,
Gritschneder, Freiherr von Wittgenstein
Postfach 86 01 09, D-8000 München 86 (DE)

## (54) Process for preparing carboxyalkyl dipeptides.

(57) A process is disclosed for preparing a carboxyalkyl dipeptide compound which comprises:

(a) protecting a peptide with a protecting group which is an α-fluorinated acyl group represented by the general formula:

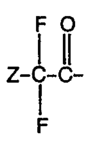

A

wherein:
Z is, e.g., $C_1$–$C_{12}$ alkyl, aryl of $C_6$ or $C_{10}$, or heterocyclic groups of up to $C_7$ containing an O, N or S heteroatom;

(b) coupling said protected peptide or amino acid with another peptide or amino acid to obtain a protected dipeptide;

(c) reductively alkylating said protected dipeptide with a keto acid or keto ester; and,

(d) hydrolyzing said reductively alkylated dipeptide to simultaneously remove said protecting group therefrom and recover said carboxyalkyl dipeptide compound.

The protecting groups employed in this process permit them to be removed concurrently with obtaining the desired dipeptide compounds thereby avoiding the need for step-wise removal of such groups in separate reactions before the desired dipeptide compounds can be obtained.

## TITLE OF THE INVENTION

PROCESS FOR PREPARING CARBOXYALKYL DIPEPTIDES

## BACKGROUND OF THE INVENTION

Typical processes for preparing carboxyalkyl dipeptides and derivatives thereof are disclosed in U.S. Patent 4,374,829. While these processes are adequate, they are not commercially attractive since the protecting groups employed therein are costly, relatively unstable and require additional, separate, reactions for their step-wise removal. Illustrative of these prior art protecting groups are such compounds as N-formyl, N-t-butoxycarbonyl, N-carbobenzyloxy, phthaloyl, succinoyl, and the like. Furthermore, the use of such protecting groups results in more process steps and, consequently, longer and costlier processing conditions.

SUMMARY OF THE INVENTION

It has now been found that carboxyalkyl dipeptides of the type disclosed in U.S. Patent 4,374,829 can be obtained at reduced costs and under shorter reaction conditions by employing the improved process of the present invention. The process of the present invention comprises the use of protecting groups that are less expensive, relatively more stable than those typically used in prior art processes, and which can be simultaneously and easily removed as the desired end product is obtained thereby avoiding the need for separate, time-consuming and costly reaction steps.

DETAILED DESCRIPTION OF THE INVENTION

In the process of the present invention, the protecting groups employed are α-fluorinated acyl groups represented by the general formula:

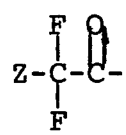

A

wherein:

Z is unsubstituted or substituted $C_1$-$C_{12}$ alkyl wherein the substituent can be halo (F, Br, Cl, I), lower $C_1$-$C_8$ alkoxy, aryloxy wherein the aryl is $C_6$ or $C_{10}$, dilower $C_1$-$C_8$ alkyl, lower $C_1$-$C_8$ alkylthio, $C_6$ or $C_{10}$ arylthio; unsubstituted or substituted aryl of $C_6$ or $C_{10}$ wherein the substituent can be lower

$C_1$-$C_8$ alkyl, lower $C_1$-$C_8$ alkoxy, or
halo (F, Br, Cl, I);
heterocyclic groups of up to $C_7$ containing
an O, N or S heteroatom.

A preferred protecting group is trifluoroacetyl (TFA).

The carboxyalkyl dipeptides which can be obtained by the process of the present invention are those disclosed in U.S. Patent 4,374,829 which is incorporated herein by reference thereto. These carboxyalkyl dipeptide compounds are represented by the formula:

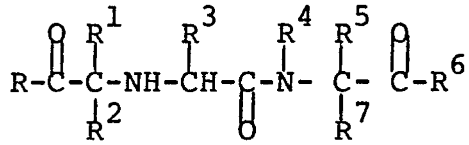

I

wherein

R and $R^6$ are the same or different and are hydroxy,
lower $C_1$-$C_8$ alkoxy
lower $C_1$-$C_8$ alkenoxy,
dilower $C_1$-$C_8$ alkylamino lower $C_1$-$C_8$ alkoxy (dimethylaminoethoxy),
acylamino lower $C_1$-$C_8$ alkoxy (acetylaminoethoxy),
acyloxy lower $C_1$-$C_8$ alkoxy (pivaloyloxymethoxy),
aryloxy, such as phenoxy,
aryllower $C_1$-$C_8$ alkoxy, such as benzyloxy,
substituted aryloxy or substituted aryllower $C_1$-$C_8$ alkoxy wherein the substituent is methyl, halo or methoxy,

amino,
lower $C_1$-$C_8$ alkylamino,
di(lower $C_1$-$C_8$ alkyl)amino,
hydroxyamino,
aryl lower $C_1$-$C_8$ alkylamino such as benzylamino;

$R^1$ is hydrogen,
hydrocarbon of from 1 to 20 carbon atoms which include branched and unsaturated (such as allyl) groups,
$C_3$-$C_{10}$ cycloalkyl;
substituted lower $C_1$-$C_8$ alkyl wherein the substituent can be halo, hydroxy, lower $C_1$-$C_8$ alkoxy;
aryloxy such as phenoxy;
amino;
di(lower $C_1$-$C_8$ alkyl)amino;
acylamino such as acetamido and benzamido,
arylamino;
guanidino;
imidazolyl;
indolyl;
mercapto;
lower $C_1$-$C_8$ alkylthio;
arylthio such as phenylthio;
carboxy or carboxamido;
carbo(lower $C_1$-$C_8$) alkoxy;
aryl such as phenyl or naphthyl;
substituted aryl such as phenyl wherein the substituent is lower $C_1$-$C_8$ alkyl, lower $C_1$-$C_8$ alkoxy or halo,
aryl lower $C_1$-$C_8$ alkyl, aryl lower $C_2$-$C_8$ alkenyl, heteroaryl lower $C_1$-$C_8$ alkyl or heteroaryl lower

$C_2$-$C_8$ alkenyl such as benzyl, styryl or indolyl ethyl,

substituted aryl lower $C_1$-$C_8$ alkyl, substituted aryl lower $C_1$-$C_8$ alkenyl, substituted heteroaryl lower $C_2$-$C_8$ alkyl, or substituted heteroaryl lower $C_2$-$C_8$ alkenyl, wherein the substituents are halo, dihalo, lower $C_1$-$C_8$ alkyl, hydroxy, lower $C_1$-$C_8$ alkoxy, amino, aminomethyl, acylamino (acetylamino or benzoylamino) di(lower $C_1$-$C_8$ alkyl)amino, lower $C_1$-$C_8$ alkylamino, carboxyl, halo lower $C_1$-$C_8$ alkyl, cyano or sulfonamido;

aryl lower $C_1$-$C_8$ alkyl or heteroaryl lower $C_1$-$C_8$ alkyl substituted on the alkyl portion by amino or acylamino (acetylamino or benzoylamino);

$R^2$ and $R^7$ are the same or different and are hydrogen or lower $C_1$-$C_8$ alkyl;

$R^3$ is aminomethyl phenyl lower $C_1$-$C_8$ alkyl;

amino lower $C_1$-$C_8$ alkyl;

guanidino lower $C_1$-$C_8$ alkyl;

imidazolyl lower $C_1$-$C_8$ alkyl;

indolyl lower $C_1$-$C_8$ alkyl;

mercapto lower $C_1$-$C_8$ alkyl;

monoalkylamino lower alkyl wherein the alkyl groups are $C_1$-$C_8$;

$R^4$ is hydrogen or lower $C_1$-$C_8$ alkyl;

$R^5$ is hydrogen, lower $C_1$-$C_8$ alkyl, phenyl, phenyl lower $C_1$-$C_8$ alkyl, hydroxyphenyl lower $C_1$-$C_8$ alkyl, hydroxy lower $C_1$-$C_8$ alkyl, amino lower $C_1$-$C_8$ alkyl, guanidino lower $C_1$-$C_8$ alkyl, imidazolyl lower $C_1$-$C_8$ alkyl, indolyl lower $C_1$-$C_8$ alkyl, mercapto lower $C_1$-$C_8$ alkyl or lower $C_1$-$C_8$ alkyl thio lower $C_1$-$C_8$ alkyl; or,

$R^4$ and $R^5$ may be connected together to form an alkylene bridge of from 2 to 4 carbon atoms, an alkylene bridge of from 2 to 3 carbon atoms and one sulfur atom, an alkylene bridge of from 3 to 4 carbon atoms containing a double bond or an alkylene bridge as above substituted with hydroxy, lower $C_1$-$C_8$ alkoxy, lower $C_1$-$C_8$ alkyl or dilower $C_1$-$C_8$ alkyl.

In the foregoing Z and R-$R^7$ groups, "acyl" denotes a group derived from an organic acid of up to $C_6$; "aryl" and the prefix "ar" denote aromatic rings of $C_5$-$C_{10}$; and "hetero" denotes one of the O, N, or S heteroatoms.

The process of the present invention is applicable to obtain such Formula I compounds or similar compounds which contain peptides comprised of amino acids which are members selected from the group consisting of alanine, arginine, asparagine, aspartic acid, cysteine, cystine, glutamic acid, glutamine, glycine, histidine, isoleucine, leucine, lysine,

methionine, ornithine, phenylalanine, proline, serine, threonine, tryptophan, tyrosine, and/or valine.

Of the above Formula I compounds, those of particular interest are

(a) N-(1-carboxy-3-phenylpropyl)-L-lysyl-L-proline and acid salts thereof;

(b) N-(1-carboxy-5-aminopentyl)-L-alanyl-L-proline;

(c) N-α-(1-carboxy-3-phenylpropyl)-L-lysyl-L-proline;

(d) N-α-(1-ethoxycarbonyl-3-phenylpropyl)-L-lysyl-L-proline;

(e) N-α-[1-carboxy-3-(3-indolyl)propyl]-L-lysyl-L-proline;

(f) N-α-[1-carboxy-3-(4-chlorophenyl)propyl]-L-lysyl-L-proline;

(g) N-α-[-1-carboxy-2-phenylthioethyl]-L-lysyl-L-proline;

(h) N-α-[1-carboxy-3-(4-chlorophenyl)propyl]-L-lysyl-trans-4-methoxy-L-proline;

(i) N-α-[1-carboxy-5-aminopentyl]-L-lysyl-L-proline;

(j) N-α-(1-carboxy-3-phenylpropyl)-L-ornithyl-L-proline;

(k) N-[1-ethoxycarbonyl-3-(4-imidazolyl)propyl]-L-alanyl-L-proline;

(l) N-[1-carboxy-3-(4-imidazolyl)propyl]-L-lysyl-L-proline;

(m) N-α-[1(S)-carboxy-3-phenylpropyl]-L-lysyl-L-proline; and,

(n) N-α-[1(S)-ethoxycarbonyl-3-phenylpropyl]-L-lysyl-L-proline.

In general, the process of the present invention comprises preparing a peptide or amino acid which is protected with a protecting group of the

process of the invention; coupling said protected peptide or amino acid with another peptide or amino acid to obtain a protected dipeptide; reductively alkylating said protected dipeptide with a keto acid (or ester, or amide, or hydroxamide thereof); and, hydrolyzing said reductively alkylated dipeptide to simultaneously remove the protecting group therefrom and afford the desired carboxyalkyl dipeptide compound.

The process of the invention is further illustrated in the following Reaction Scheme.

REACTION SCHEME

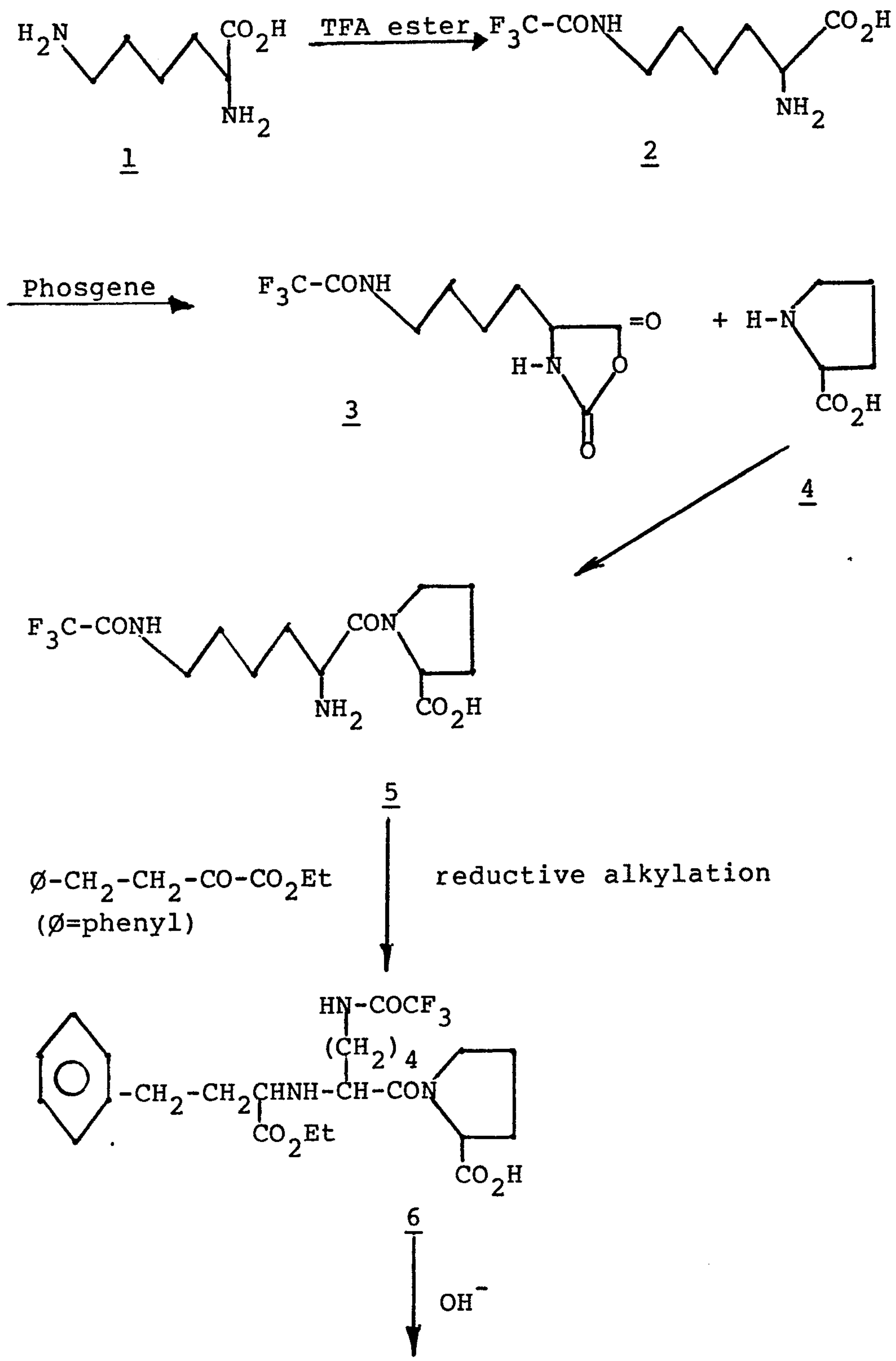


1 2 3 4 5 6

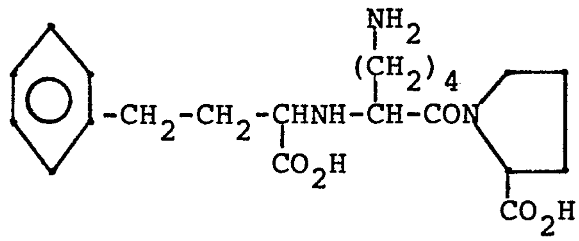

I

As shown in the Reaction Scheme, L-lysine (1) is treated with an ester of trifluoroacetic acid (a TFA ester) to obtain TFA-L-lysine 2; i.e., $N_\varepsilon$-trifluoroacetyl-L-lysine. TFA-L-lysine is then treated with phosgene to obtain the $N_\varepsilon$-trifluoroacetyl-L-lysine-N-carboxyanhydride (compound 3, TFA-lysineNCA) which, when coupled with L-proline (4), affords protected dipeptide 5; i.e., N -TFA-L-lysyl-L-proline. Protected dipeptide 5 is reductively alkylated with a suitable α-keto ester to obtain $N_\varepsilon$-trifluoroacetyl-$N_\alpha$-[(S)-α-carboxyalkyl]-L-lysyl-L-proline (6). Hydrolysis of 6 in the presence of a base yields the $N_\alpha$-carboxyalkyl-L-lysyl-L-proline dipeptide Compound I.

The process of the invention is further demonstrated in the following Examples wherein, unless indicated otherwise, all parts and percentages are by weight and all temperatures are in degrees Celsius.

EXAMPLE 1

$N_{\varepsilon}$-Trifluoroacetyl-L-lysyl-L-proline (5)

A mixture of L-proline (10.1 g), potassium hydroxide (4.89 g), potassium carbonate (17.3 g), tetrahydrofuran (THF) (250 ml) and water (325 ml) was prepared and cooled to 0°C. To this stirred mixture was rapidly added a solution of $N_{\varepsilon}$-trifluoroacetyl-L-lysine-N-carboxyanhydride (3) (22.3 g) in THF (90 ml). The pH of the resulting mixture was adjusted to 5.8 with sulfuric acid. By high pressure liquid chromatography (HPLC) assay the yield of $N_{\varepsilon}$-trifluoroacetyl-L-lysyl-L-proline (TFA-lysylproline) (5) was 26.6 g (94%). This was a noncrystalline product which was characterized as its N,N-dicyclohexylamine salt.

EXAMPLE 2

$N_{\varepsilon}$-Trifluoroacetyl-L-lysyl-L-proline Dicyclohexylamine Salt

An aqueous solution at pH 5.8 containing $N_{\varepsilon}$-trifluoroacetyl-L-lysyl-L-proline (5) (8 g) and potassium sulfate (6.2 g) was frozen and lyophilized under vacuum. The solid residue was stirred with 2-propanol (50 ml) and insoluble potassium sulfate was filtered away. To the filtrate was added N,N-dicyclohexylamine (4.27 g). The solution was seeded and crystallized at 5°C over three days. The crystalline product was washed with cold 2-propanol (15 ml) and was dried under vacuum to afford 9.13 g of compound.

M.p. 147-149°C (dec.).

Calculated for $C_{13}H_{20}F_3N_3O_4 \cdot C_{12}H_{23}N$:

C, 57.67; H, 8.32; F, 10.95; N, 10.76.

Found: C, 56.43; H, 8.95; F, 10.78; N, 10.44.

The purity as determined by HPLC was greater than 99%.

EXAMPLE 3

$N_\alpha$-[1-(S)-Ethoxycarbonyl-3-phenylpropyl]-$N_\varepsilon$-trifluoroacetyl-L-lysyl-L-proline (6)

A solution of TFA-lysylproline (73.6 g) in water and THF was concentrated under vacuum to 200 mL. 1-Butanol (200 ml) was added and the mixture was concentrated to 200 ml. This concentration procedure was repeated twice. Salts were then removed by filtration and the salt cake was washed with ethanol (400 ml). To the combined alcoholic filtrate there was added additional ethanol (one liter), Raney nickel (7.5 teaspoons), powdered 3A molecular sieves (200 g), and ethyl 2-oxo-4-phenylbutyrate (68.2 g). This slurry was agitated under 40 psi of hydrogen at 25°C. When hydrogen uptake was complete, the solids were filtered and washed with ethanol (500 ml). The combined filtrate was concentrated to a low volume and water was added (500 ml) followed by methylene chloride (500 ml). The pH was adjusted to 9.2 with sodium hydroxide and the methylene chloride layer was discarded. The water phase was extracted twice more with $CH_2Cl_2$ and these extracts were also discarded. The pH of the water layer was adjusted to 4.6 with hydrochloric acid and extracted with methylene chloride. The extract was dried with sodium sulfate and then concentrated to an oil. The oil was dissolved in methyl t-butyl ether (650 ml)

and the desired product crystallized over several hours. The slurry was diluted very slowly with cyclohexane (240 ml), stirred for several hours, and filtered to afford $N_\alpha$-[1-(S)-ethoxycarbonyl-3-phenylpropyl]-$N_\epsilon$-trifluoroacetyl-L-lysyl-L-proline (6), 54.8 g, m.p. 64-70°C.

A second crop of product, 13.9 g, was obtained from the concentrated liquors. Recrystallization of 10 grams from methyl t-butyl ether afforded a recovery of 9.1 grams, m.p. 74-77°C, shown to be isomerically pure by HPLC.

Calculated for $C_{25}H_{34}F_3N_3O_6$:
C, 56.70; H, 6.47; N, 7.94.
Found: C, 56.73; H, 6.54; N, 7.75.
Specific rotation, $[\alpha]_D^{25°C}$=-24.7° (0.1N HCl/$CH_3OH$)
$[\alpha]_{405nm}^{25°C}$=-53.8° (0.1N HCl/$CH_3OH$)

## EXAMPLE 4

$N_\alpha$-[1-(S)-Carboxy-3-phenylpropyl]-L-lysyl-L-proline (I)

$N_\alpha$-[1-(S)-Ethoxycarbonyl-3-phenylpropyl]-$N_\epsilon$-trifluoroacetyl-L-lysyl-L-proline (6) (10 g) was stirred in water (60 ml) with sodium hydroxide (6.05 g) for about three hours. The pH was adjusted to 2.0 with concentrated hydrochloric acid and the solution was placed on a strong acid (sulfonic acid type) ion exchange resin on the acid cycle. Once the product was adsorbed, the column was washed with water to remove salts. The product was eluted from the ion exchange resin using 4% pyridine in water. The eluates were combined and concentrated to about 100 ml and the pH was adjusted to 5.2 with hydrochloric acid. The solution was concentrated to about 16 grams, and the concentrate diluted with

ethanol (140 ml). The resulting solution was concentrated to about 22 g, and rediluted with ethanol (about 30 ml) until the water content was about 8 to 12% (80 to 120 mg of water per milliliter of solution). The product was allowed to crystallize slowly. It was then filtered, washed with 9:1 ethanol-water, and dried to afford 7.6 to 7.9 g (90-94%) of $N_\alpha$-[1-(S)-carboxy-3-phenylpropyl]-L-lysyl-L-proline dihydrate.

Percent water measured by Karl Fischer reagent: 8.2%.

Specific rotation, $[\alpha]_D^{25}$ = -26.8° (0.1N HCl in $CH_3OH$).

<u>WHAT IS CLAIMED IS</u>:

1. A process for preparing a carboxyalkyl dipeptide compound which comprises:

(a) protecting a peptide with a protecting group which is an α-fluroinated acyl group represented by the general formula:

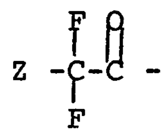

<u>A</u>

wherein:

Z is unsubstituted or substituted $C_1$-$C_{12}$ alkyl wherein the substituent can be halo, lower $C_1$-$C_8$ alkoxy, aryloxy wherein the aryl is $C_6$ or $C_{10}$, di(lower $C_1$-$C_8$ alkyl), lower $C_1$-$C_8$ alkylthio, aryl $C_6$ or $C_{10}$ thio;

unsubstituted or substituted aryl of $C_6$ or $C_{10}$ wherein the substituent can be lower $C_1$-$C_8$ alkyl, lower $C_1$-$C_8$ alkoxy, or halo;

heterocyclic groups of up to $C_7$ containing an O, N or S heteroatom;

(b) coupling said protected peptide or amino acid with another peptide or amino acid to obtain a protected dipeptide;

(c) reductively alkylating said protected dipeptide with a keto acid or keto ester; and,

(d) hydrolyzing said reductively alkylated dipeptide to simultaneously remove said protecting group therefrom and recover said carboxyalkyl dipeptide compound.

2. The process of Claim 1 wherein said protecting group is trifluoracetyl.

3. The process of Claim 1 wherein said peptide is comprised of amino acids which are members selected from the group consisting of alanine, arginine, asparagine, aspartic acid, cysteine, cystine, glutamic acid, glutamine, glycine, histidine, isoleucine, leucine, lysine, methionine, ornithine, phenylalanine, proline, serine, threonine, tryptophan, tyrosine, and/or valine.

4. The process of Claim 1 wherein said carboxyalkyl dipeptide compound has the general formula:

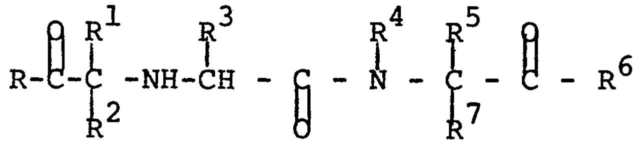

I

wherein

R and $R^6$ are the same or different and are hydroxy,
lower $C_1$-$C_8$ alkoxy;
lower $C_2$-$C_8$ alkenoxy;
di(lower $C_1$-$C_8$ alkyl)amino lower $C_1$-$C_8$ alkoxy;
acylamino lower $C_1$-$C_8$ alkoxy;
acyloxy lower $C_1$-$C_8$ alkoxy;

aryloxy;
aryl lower $C_1$-$C_8$ alkoxy;
substituted aryloxy or substituted
aryl lower $C_1$-$C_8$ alkoxy wherein the
substituent is methyl, halo or methoxy;
amino;
lower $C_1$-$C_8$ alkylamino;
di(lower $C_1$-$C_8$ alkyl)amino;
hydroxyamino;
aryl lower $C_1$-$C_8$ alkylamino;

$R^1$ is hydrogen;
hyrocarbon of from 1 to 20 carbon atoms which include branched and unsaturated groups;
$C_3$-$C_{10}$ cycloalkyl;
substituted lower $C_1$-$C_8$ alkyl wherein
the substituent can be halo, hydroxy, lower
$C_1$-$C_8$ alkoxy;
aryloxy;
amino;
di(lower $C_1$-$C_8$ alkyl)amino;
acylamino;
arylamino;
guanidino;
imidazolyl;
indolyl;
mercapto;
lower $C_1$-$C_8$ alkylthio;
arylthio;
carboxy or carboxamido;
carbolower $C_1$-$C_8$ alkoxy;
aryl;
substituted aryl wherein the

substituent is lower $C_1$-$C_8$ alkyl, lower $C_1$-$C_8$ alkoxy or halo;
aryl lower $C_1$-$C_8$ alkyl;
aryl lower $C_2$-$C_8$ alkenyl;
heteroaryllower $C_1$-$C_8$ alkyl or heteroaryl lower $C_2$-$C_8$ alkenyl;
substituted aryl lower $C_1$-$C_8$ alkyl, substituted aryl lower $C_2$-$C_8$ alkenyl, substituted heteroaryl lower $C_1$-$C_8$ alkyl, or substituted heteroaryl lower $C_2$-$C_8$ alkenyl, wherein the substituent(s) is halo, dihalo, lower $C_1$-$C_8$ alkyl, hydroxy, lower $C_1$-$C_8$ alkoxy, amino, aminomethyl, acylamino di(lower $C_1$-$C_8$ alkyl)amino, lower $C_1$-$C_8$ alkylamino, carboxyl, halo lower $C_1$-$C_8$ alkyl, cyano or sulfonamido;
aryl lower $C_1$-$C_8$ alkyl or heteroaryl lower $C_1$-$C_8$ alkyl substituted on the alkyl portion by amino or acylamino;

$R^2$ and $R^7$ are the same or different and are hydrogen;
or lower $C_1$-$C_8$ alkyl;

$R^3$ is aminomethyl phenyl lower $C_1$-$C_8$ alkyl;
amino lower $C_1$-$C_8$ alkyl;
guanidino lower $C_1$-$C_8$ alkyl;
imidazolyl $C_1$-$C_8$ lower alkyl;
indolyl lower $C_1$-$C_8$ alkyl;
mercapto lower $C_1$-$C_8$ alkyl;
monoalkylamino lower alkyl wherein the alkyl groups are $C_1$-$C_8$;

$R^4$ is hydrogen or lower $C_1$-$C_8$ alkyl;

$R^5$ is hydrogen; lower $C_1$-$C_8$ alkyl; phenyl; phenyl lower $C_1$-$C_8$ alkyl; hydroxy phenyl lower $C_1$-$C_8$ alkyl; hydroxy lower $C_1$-$C_8$ alkyl; amino lower $C_1$-$C_8$ alkyl; guanidino lower $C_1$-$C_8$ alkyl; imidazolyl lower $C_1$-$C_8$ alkyl; indolyl lower $C_1$-$C_8$ alkyl; mercapto lower $C_1$-$C_8$ alkyl; lower $C_1$-$C_8$ alkyl thio lower $C_1$-$C_8$ alkyl; or,

$R^4$ and $R^5$ may be connected together to form an alkylene bridge of from 2 to 4 carbon atoms; an alkylene bridge of from 2 to 3 carbon atoms and one sulfur atom; an alkylene bridge of from 3 to 4 carbon atoms containing a double bond; or an alkylene bridge as above substituted with hydroxy, lower $C_1$-$C_8$ alkoxy, lower $C_1$-$C_8$ alkyl or di(lower $C_1$-$C_8$ alkyl).

5. The process of Claim 4 wherein said carboxyalkyl dipeptide compound is a member selected from the group consisting of:

(a) N-(1-carboxy-3-phenylpropyl)-L-lysyl-L-proline and acid salts thereof;

(b) N-(1-carboxy-5-aminophenyl)-2-alanyl-L-proline;

(c) $N_\alpha$-(1-carboxy-3-phenylpropyl)-L-lysyl-L-proline;

(d) $N_\alpha$-(1-ethoxycarbonyl-3-phenylpropyl)-L-lysyl-L-proline;

(e) $N_{\alpha}$-[1-carboxy-3-(3-indolyl)propyl]-L-lysyl-L-proline;

(f) $N_{\alpha}$-[1-carboxy-3-(4-chlorophenyl)propyl]-L-lysyl-L-proline;

(g) $N_{\alpha}$-[1-carboxy-2-phenylthioethyl]-L-lysyl-L-proline;

(h) $N_{\alpha}$-[1-carboxy-3-(4-chlorophenyl)propyl]-L-lysyl-trans-4-methoxy-L-proline;

(i) $N_{\alpha}$-[1-carboxy-5-aminopentyl]-L-lysyl-L-proline;

(j) $N_{\alpha}$-(1-carboxy-3-phenylpropyl)-L-ornithyl-L-proline;

(k) N-[1-ethoxycarbonyl-3-(4-imidazolyl)propyl]-L-alanyl-L-proline;

(l) N-[1-carboxy-3-(4-imidazolyl)propyl]-L-lysyl-L-proline;

(m) N-α-[1(S)-carboxy-3-phenylpropyl]-L-lysyl-L-proline; and,

(n) N-α-[1(S)-ethoxycarbonyl-3-phenylpropyl]-L-lysyl-L-proline.

6. A compound having the formula:

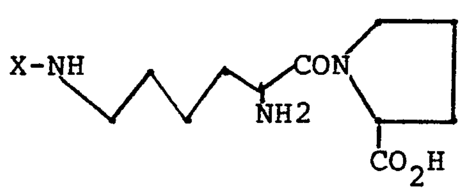

5

wherein X is:

an α-fluorinated acyl protecting group represented by the genral formula:

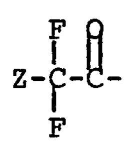

A

wherein:

Z is unsubstituted or substituted $C_1$-$C_{12}$ alkyl wherein the substituent can be halo, lower $C_1$-$C_8$ alkoxy, aryloxy wherein the aryl is $C_6$ or $C_{10}$, dilower $C_1$-$C_8$ alkyl, lower $C_1$-$C_8$ alkylthio, aryl $C_6$ or $C_{10}$ thio; unsubstituted or substituted aryl of $C_6$ or $C_{10}$ wherein the substituant can be lower $C_1$-$C_8$ alkyl, lower $C_1$-$C_8$ alkoxy, or halo; heterocyclic groups of up to $C_7$ containing an O, N or S heteroatom.

7. The compound of Claim 6 wherein X is

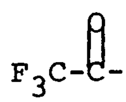

8. A compound having the formula:

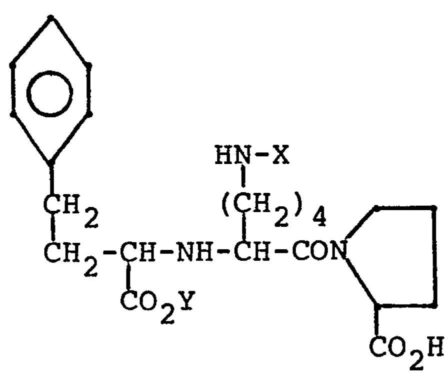

6

wherein X is:

an α-fluorinated acyl protecting group represented by the genral formula:

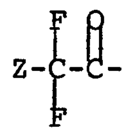

A

wherein:

Z is unsubstituted or substituted $C_1$-$C_{12}$ alkyl wherein the substituent can be halo, lower $C_1$-$C_8$ alkoxy, aryloxy wherein the aryl is $C_6$ or $C_{10}$, dilower $C_1$-$C_8$ alkyl, lower $C_1$-$C_8$ alkylthio, aryl $C_6$ or $C_{10}$ thio; unsubstituted or substituted aryl of $C_6$ or $C_{10}$ wherein the substituant can be lower $C_1$-$C_8$ alkyl, lower $C_1$-$C_8$ alkoxy, or halo; heterocyclic groups of up to $C_7$ containing an O, N or S heteroatom; and,

Y is unsubstitated or substitated $C_1$-$C_{12}$ alkyl wherein the substituent can be halo, hydroxy, $C_1$-$C_8$ alkoxy; unsubstituted or substituted $C_6$-$C_{10}$ aryl wherein the substituent can be hydroxy, $C_1$-$C_8$ alkyl, $C_1$-$C_8$ alkoxy.

9. The compound of Claim 8 wherein X is